# EUROPEAN PATENT APPLICATION

(11) **EP 4 768 590 A1**
(43) Date of publication of application: **01.07.2026**
(21) Application number: 25786948.7
(22) Date of filing: 11.04.2025
(51) Int. Cl.: C12N 15/77, C07K 14/245, C12P 13/08

(54) **MICROORGANISM INCLUDING PROTEIN ENCODED BY FOREIGN FTSN GENE AND METHOD FOR PRODUCING L-AMINO ACID USING SAME**

(30) Priority: 12.04.2024 KR 20240049295
(71) Applicant: CJ Cheiljedang Corporation, Seoul 04560 (KR)
(72) Inventor: KIM, Hye Mi, Seoul 04560 (KR); SHIN, Kwang Soo, Seoul 04560 (KR); CHOI, Woosung, Seoul 04560 (KR)
(74) Representative: Meissner Bolte Partnerschaft mbB
(86) International application number: PCT/KR2025/004923
(87) International publication number: WO 2025/216576

(57) **Abstract**

The present disclosure relates to a microorganism including a protein encoded by a foreign ftsN gene, and a method for producing an L-amino acid using same.

## Description

### [TECHNICAL FIELD]

### Cross-reference to prior application(s)

The present disclosure claims the benefit of the priority based on Korean Patent Application No. 10-2024-0049295 filed on April 12, 2024, and the entire contents disclosed in the document of the corresponding Korean patent application are incorporated as a part of the present disclosure.

The present disclosure relates to a microorganism into which a protein encoded by a foreign *ftsN* gene is introduced and a method for producing L-amino acids using the same.

### [BACKGROUND ART]

Microorganisms of the genus *Corynebacterium* are gram-positive microorganisms widely used in production of L-amino acids. L-amino acids, especially, L-lysine, are used in not only animal feed but also human pharmaceutical and cosmetic industries, and are mainly produced through fermentation using *Corynebacterium* strains.

Until now, many attempts have been made to improve the production ability of L-lysine using a strain of the genus *Corynebacterium.* Among them, there is research to improve a Corynebacterium strain producing L-amino acids by disrupting or attenuated expressing a specific gene using a recombinant DNA technology. In addition, research has been conducted to study the effect on L-lysine production by amplifying each gene related to L-lysine biosynthesis and improve L-amino acid-producing Corynebacterium strains.

On the other hand, in the lysine production industry through fermentation, high concentration lysine production and increased lysine production ability of microorganism are important factors. Therefore, there have been efforts to improve the lysine production ability of microorganisms and continuously maintain it during fermentation culture. However, there is a problem that it is difficult to maintain lysin production ability until the latter half of culture due to various internal or external factors which inhibit the activity of microorganisms.

Therefore, the development of a strain with improved and continuously maintainable production ability of L-amino acids, for example, L-lysine, is being required.

### [DISCLOSURE]

### [TECHNICAL PROBLEM]

One object of the present disclosure is to provide a microorganism of the genus *Corynebacterium,* into which a protein encoded by a foreign *ftsN* gene is introduced. The foreign *ftsN* gene may be derived from a microorganism of the genus *Escherichia,* for example, *Escherichia coli.*

Another object of the present disclosure is to provide a composition for producing a target product (for example, L-amino acid) comprising the microorganism.

Other object of the present disclosure is to provide a method for production of a target product (for example, L-amino acid) comprising culturing the microorganism in a medium.

### [TECHNICAL SOLUTION]

This is explained in detail as follows. On the other hand, each description and embodiment disclosed in the present application can also be applied to each other description and embodiment. In other words, all combinations of the various elements disclosed in the present application belong to the scope of the present application. In addition, the scope of the present application cannot be considered limited by the specific description described below. Furthermore, those skilled in the art can recognize or confirm many equivalents to the specific embodiments of the present application described in the present application using only a common experiment. In addition, these equivalents are intended to be incorporated in the present application.

One embodiment of the present disclosure provides a microorganism into which a protein encoded by a foreign *ftsN* gene is introduced.

The microorganism may be a microorganism producing a target product.

In the present disclosure, the target product may be selected from the group consisting of amino acids (for example, L-amino acids), organic acids, and combinations thereof. The term "amino acid" or "L-amino acid" generally refers to a basic configuration unit of protein composing the body of living organisms, in which an amino group and a carboxyl group are bonded to the same carbon atom. The amino acid may be selected from the group consisting of glycine, alanine, valine, leucine, isoleucine, threonine, serine, cysteine, cystine, methionine, aspartic acid, asparagine, glutamic acid, diiodotyrosine, lysine, arginine, histidine, phenylalanine, tyrosine, tryptophan, proline, oxyproline, and combinations thereof, but not limited thereto. The term "organic acid" may be an acidic organic compound, specifically, an organic compound including a carboxyl group and a sulfone group. As an example of the organic acid, lactate, acetate, succinate, butyrate, palmitate, oxalate, tartaric acid, citrate, tartrate, propionate, hexenoic acid, capric acid, caprylic acid, valeric acid, or citric acid, but not limited thereto. In one specific embodiment, the amino acid may be L-lysine, but not limited thereto.

In the present disclosure, the term, *"ftsN* gene" refers to a gene encoding cell division protein FtsN protein.

In the present disclosure, the term, "cell division protein FtsN" means a protein involved in cell division by activating synthesis and contraction of cellular septum peptidoglycan. In the present disclosure, the cell division protein FtsN may be interchangeably used with "FtsN protein". In the present disclosure, the sequence of the "cell division protein FtsN" may be obtained from a known database, GenBank of NCBI (for example, NCBI Reference Sequence: NP_418368.1). Specifically, the cell division protein FtsN may be a protein encoded by the *ftsN* gene.

As one example, the foreign *ftsN* gene of the present disclosure may be a gene of a different origin from a microorganism of the genus *Corynebacterium* into which the gene is introduced, or may be different from a gene endogenously present in the microorganism of the genus *Corynebacterium* into which the gene is introduced. Specifically, the foreign *ftsN* gene may be derived from a microorganism of the genus *Escherichia,* for example, derived from *Escherichia coli,* but not limited thereto, and on the purpose of the present disclosure, any one may be comprised in the gene without limitation as long as it can intensify production ability of a target product, specifically, L-amino acid, for example, L-lysine. The sequence of the *ftsN* gene may be obtained from a known database, GenBank of NCBI, and to the method for securing the corresponding sequence, various methods well known in the art may be applicable.

In the present disclosure, the protein encoded by the foreign *ftsN* gene may have or comprise the amino acid sequence of SEQ ID NO: 9, or consist of the amino acid sequence, or be essentially consisting of the amino acid sequence.

In one embodiment, the protein encoded by the foreign *ftsN* gene may comprise an amino acid sequence having sequence identity or homology of at least 70% or more, 75% or more, 80% or more, 85% or more, 90% or more, 91% or more, 92% or more, 93% or more, 94% or more, 95% or more, 96% or more, 97% or more, 98% or more, 99% or more, 99.5% or more, 99.7% or more, or 99.9% or more and less than 100% to the amino acid sequence of SEQ ID NO: 9, or consist of the sequence. In addition, as long as it is an amino acid sequence having such homology or identity and showing activity corresponding to cell division protein FtsN, variants having an amino acid sequence in which some sequences are deleted, modified, substituted, conservatively substituted or added may be comprised in the protein encoded by the foreign *ftsN* gene. For example, it may be a case having sequence addition or deletion, mutation that may occur naturally, silent mutation, or conservative substitution, which does not alter the cell division protein at the N-terminus, C-terminus and/or inside of the amino acid sequence.

The "conservative substitution" means substituting one amino acid with another amino acid having similar structural and/or chemical properties. This amino acid substitution may occur generally based on the similarity in polarity, charge, solubility, hydrophobicity, hydrophilicity and/or amphipathic nature of residues. In common, conservative substitution may hardly affect or not affect activity of protein or polypeptide.

The foreign *ftsN* gene of the present disclosure may have or comprise the nucleic acid sequence of SEQ ID NO: 10, or consist of the nucleic acid sequence, or be essentially consisting of the nucleic acid sequence.

In one embodiment, the foreign *ftsN* gene may comprise a sequence having sequence identity or homology of at least 70% or more, 75% or more, 80% or more, 85% or more, 90% or more, 91% or more, 92% or more, 93% or more, 94% or more, 95% or more, 96% or more, 97% or more, 98% or more, 99% or more, 99.5% or more, 99.7% or more, or 99.9% or more and less than 100% to the nucleic acid sequence of SEQ ID NO: 10, or consist of the sequence.

In the present disclosure, that a polynucleotide or polypeptide "has, or comprises a specific nucleic acid sequence (base sequence) or an amino acid sequence, or consists of the sequence, or be essentially consisting of the sequence" may refer to that the polynucleotide or polypeptide essentially comprises the specific nucleic acid sequence (base sequence) or amino acid sequence, and it may be interpreted to include "a substantially equivalent sequence" in which a mutation (deletion, substitution, modification, and/or addition) is applied to the specific nucleic acid sequence (base sequence) or amino acid sequence within a range of maintaining the original function and/or desired function of the polynucleotide or polypeptide (or not exclude the mutation). In one embodiment, that a polynucleotide or polypeptide "has or comprises a specific nucleic acid sequence (base sequence) or amino acid sequence" may refer to that the polynucleotide or polypeptide (i) essentially comprises the specific nucleic acid sequence (base sequence) or amino acid sequence, or (ii) consists of a nucleic acid sequence or an amino acid sequence having homology or identity of 70% or more, 80% or more, 85% or more, 90% or more, 91% or more, 92% or more, 93% or more, 94% or more, 95% or more, 96% or more, 97% or more, 98% or more, 98% or more, 99% or more, 99.5% or more, or 99.9% or more to the specific nucleic acid sequence (base sequence) or amino acid sequence, or essentially comprises thereof, and maintain the original function and/or desired function.

In one embodiment, the desired function may mean a function of giving production ability of a target product, specifically, L-amino acid, for example, L-lysine, or increasing (improving) thereof into a microorganism.

In the present disclosure, 'homology' or 'identity' means the degree of similarity between two given amino acid sequences or base sequences, and may be expressed as a percentage (%). The terms homology and identity may be often used interchangeably.

The sequence homology or identity of the conserved polynucleotide or polypeptide may be determined by a standard array algorithm, and a default gap penalty established by a used program may be used together. Substantially, the homologous or identical sequences may be generally hybridized with the entire or a part of the sequence under moderate or high stringent conditions. It is obvious that hybridization includes hybridization with a polynucleotide containing a common codon or a codon considering codon degeneracy in the polynucleotide.

Whether any two polynucleotide or polypeptide sequences have homology or identity, may be determined using a default parameter as in for example, Pearson et al (1988) [Proc. Natl. Acad. Sci. USA 85]: 2444 using a known computer algorithm such as "FASTA" program. Otherwise, as performed in Needleman program of EMOSS package (EMBOSS: The European Molecular Biology Open Software Suite, Rice et al., 2000, Trends Genet. 16: 276-277) (version 5.0.0 or later version), it may be determined using Needleman-Wunsch algorithm (Needleman and Wunsch, 1970, J. Mol. Biol. 48: 443-453) (including GCG program package (Devereux, J., et al, Nucleic Acids Research 12: 387 (1984)), BLASTP, BLASTN, FASTA (Atschul, [S.] [F.,] [ET AL, J MOLEC BIOL 215]: 403 (1990); Guide to Huge Computers, Martin J. Bishop, [ED.,] Academic Press, San Diego,1994, and [CARILLO ETA/.](1988) SIAM J Applied Math 48: 1073). For example, using BLAST, or ClustalW of National Center of Biotechnology Information Database, the homology or identity may be determined.

The homology or identity of the polynucleotide or polypeptide may be determined by comparing sequence information using GAP computer program such as for example, Needleman et al. (1970), J Mol Biol. 48:443, as known in for example, Smith and Waterman, Adv. Appl. Math (1981) 2:482. In summary, GAP program may be defined as the total number of symbols in the shorter of two sequences divided by the number of similarly arranged symbols (i.e., nucleotides or amino acids). The default parameters for the GAP program may comprise (1) a binary comparison matrix (containing values of 1 for identity and 0 for non-identity) and a weighted comparison matrix of Gribskov et al (1986) Nucl. Acids Res. 14: 6745, disclosed in Schwartz and Dayhoff, eds., Atlas Of Protein Sequence And Structure, National Biomedical Research Foundation, pp. 353-358 (1979) (or EDNAFULL (EMBOSS version of NCBI NUC4.4) substitution matrix); (2) a 3.0 penalty for each gap and an additional 0.10 penalty for each symbol in each gap (or gap opening penalty 10, gap extension penalty 0.5); and (3) no penalty for end gaps.

In the present disclosure, the term, "introduction of protein" refers to introducing activity of protein into a microorganism having no specific protein activity. This may be also expressed as enhancement of activity of protein in a microorganism having no specific protein activity.

The introduction of protein may be performed by introducing a foreign polynucleotide encoding a protein exhibiting the same/similar activity to the protein, or a codon-optimized mutant polynucleotide thereof into a host cell. The foreign polynucleotide may be used without limitation in its origin or sequence as long as it exhibits the same/similar activity to the protein. **In** addition, the introduced foreign polynucleotide may be introduced into a host cell by optimizing a codon thereof so that optimized transcription and translation are carried out in the host cell. The introduction may be performed by appropriately selecting a known transformation method by those skilled in the art, and as the introduced polynucleotide is expressed in the host cell, the protein is produced, so its activity can be increased.

The enhancement of the introduced protein,
may be performed by
1) increasing a copy number in cells of a polynucleotide encoding the protein,
2) modification of an expression regulatory sequence to increase expression of the polynucleotide,
3) modification of a polynucleotide sequence on chromosome to enhance the activity of the protein, or
4) a method for modification to be enhanced by a combination thereof, but is not limited thereto.

The 1) increasing a copy number of a polynucleotide, is not particularly limited thereto, but may be performed in a form operably linked to a vector, or may be performed by being inserted into chromosome in a host cell. Specifically, it may be performed by introducing a polynucleotide encoding the protein of the present application into a host cell as it is operably linked to a vector which can replicate and function independently of the host, or may be performed by a method for increasing a copy number of the polynucleotide in chromosome of the host cell by introducing the polynucleotide into a host cell as it is operably linked to a vector which can insert the polynucleotide into the chromosome in the host cell.

Next, the 2) modification of an expression regulatory sequence to increase expression of a polynucleotide, is not particularly limited thereto, but may be performed by inducing a mutation on the sequence by deletion, insertion, non-conservative or conservative substitution or a combination thereof of a nucleic acid sequence so that the activity of the expression regulatory sequence is further enhanced, or may be performed by replacing it with a nucleic acid sequence having stronger activity. The expression regulatory sequence, is not particularly limited thereto, but may comprise a promoter, an operator sequence, a sequence encoding a ribosome binding site, a sequence regulating termination of transcription and translation, and the like.

A strong heterogenous promoter may be linked to the upstream of the polynucleotide expression unit instead of the original promoter, and the example of the strong promoter includes CJ7 promoter (Korean Registered Patent No. 0620092 and WO2006/065095), lysCP1 promoter (WO2009/096689), spl1 promoter, spl7 promoter, spl13 promoter (Korean Registered Patent No. 1783170), EF-Tu promoter, groEL promoter, aceA or aceB promoter, or the like, but not limited thereto. In addition, the 3) modification of a polynucleotide sequence on chromosome, is not particularly limited thereto, but may be performed by inducing a mutation on the expression regulatory sequence by deletion, insertion, non-conservative or conservative substitution or a combination thereof of a nucleic acid sequence to further enhance the activity of the polynucleotide sequence, or may be performed by replacing it with a polynucleotide sequence improved to have stronger activity.

Lastly, the method for modification to be enhanced by a combination thereof, may be performed by applying at least one method of increasing a copy number of a polynucleotide encoding the introduced protein, modification of an expression regulatory sequence to increase its expression, modification of the polynucleotide sequence inserted into chromosome, and modification of a foreign polynucleotide exhibiting the activity of the protein or modification of a codon-optimized mutant polynucleotide thereof together.

The term "vector" of the present disclosure refers to a DNA product containing a tarter polynucleotide sequence operably linked to an appropriate regulatory sequence, so as to introduce a target gene in a suitable host. The regulatory sequence may comprise a promoter which can initiate transcription, any operator sequence to regulate such transcription, a sequence encoding an appropriate mRNA ribosome binding site, and a sequence regulating termination of transcription and translation. The vector may replicate or function regardless of host genome, and may be integrated into the genome itself, after being transformed into a suitable host cell. As one example, the target polynucleotide in chromosome may be replaced with a mutated polynucleotide by a vector for intracellular chromosome insertion. The insertion of the polynucleotide into chromosome may be conducted by any method known in the art, for example, homologous recombination, but is not limited thereto.

The vector of the present disclosure is not particularly limited, and any vector known in the art may be used. The examples of the commonly used vectors may include plasmids, cosmids, viruses, bacteriophages, and the like in a natural state or recombinant state. For example, as the phage vector or cosmid vector, pWE15, M13, MBL3, MBL4, IXII, ASHII, APII, t10, t11, Charon4A, and Charon21A and the like may be used, and as the plasmid vector, pBR-based, pUC-based, pBluescriptII-based, pGEM-based, pTZ-based, pCL-based and pET-based and the like may be used. Specifically, pDZ, pDC24, pACYC177, pACYC184, pCL, pECCG117, pUC19, pBR322, pMW118, pCC1BAC vectors and the like may be used.

The term "transformation" of the present disclosure refers to introducing a vector comprising a polynucleotide encoding a target protein into a host cell to express a protein encoded by the polynucleotide in the host cell. The transformed polynucleotide may include all as long as it can be expressed in a host cell, regardless of whether it is inserted and positioned in chromosome of the host cell or is positioned outside the chromosome. In addition, the polynucleotide comprises DNA and RNA encoding a target protein. The polynucleotide may be introduced in any form, as long as it can be introduced into a host cell and expressed. For example, the polynucleotide may be introduced in a form of an expression cassette which is a gene structure comprising all elements required for being expressed by itself into a host cell. The expression cassette may commonly comprise a promoter operably linked to the polynucleotide, a transcription termination signal, a ribosome binding site, and a translation termination signal. The expression cassette may be in a form of an expression vector capable of self-replication. In addition, the polynucleotide may be operably linked to a sequence required for expression in a host cell by being introduced in its own form into a host cell, but is not limited thereto.

In addition, the term of the present disclosure, "operably linked" means that a promoter sequence that initiates and mediates transcription of a polynucleotide encoding the target protein of the present application and the gene sequence are functionally linked.

The method for transforming the vector of the present disclosure includes any method for introducing a nucleic acid into a cell, and may be performed by selecting an appropriate standard technology as known in the art depending on the host cell. For example, there are electroporation, lipofection, calcium phosphate (CaPO₄) precipitation, calcium chloride (CaCl₂) precipitation, microinjection, polyethylene glycol (PEG) method, DEAE-dextran method, cation liposome method, and lithium acetate-DMSO method and the like, but not limited thereto.

The term "microorganism producing a target product (for example, L-amino acid)" of the present disclosure, is a wild-type microorganism or a microorganism in which genetic modification occurs naturally or artificially, and includes all microorganisms having production ability of a target product (for example, L-amino acid), and may be a microorganism in which a specific mechanism is weakened or enhanced due to causes such as insertion of an external gene, or enhancement or inactivation of activity of an endogenous gene, or the like, which is a microorganism in which genetic mutation occurs or its activity is enhanced for production of a target product (for example, L-amino acid).

For the purpose of the present disclosure, the "microorganism producing a target product (for example, L-amino acid)" may be used to mean a case in which a microorganism having production ability of a target product (for example, L-amino acid) has production ability of a target product (for example, L-amino acid) by introducing a protein encoded by a foreign *ftsN* gene (for example, E. coli-derived *ftsN* gene), and/or a case in which a microorganism having no production ability of a target product (for example, L-amino acid) has production ability of a target product (for example, L-amino acid) by introducing a protein encoded by a foreign *ftsN* gene (by being mutated to enhance/express activity of a foreign cell division protein FtsN). In the present disclosure, "microorganism" may be interchangeably used with "strain", "cell".

The target product may be selected from the group consisting of amino acids (for example, L-amino acids), organic acids and combinations thereof. The term "amino acid" or "L-amino acid" refers to a basic configuration unit of a protein composing the body of living organisms, in which an amino group and a carboxyl group are bonded to the same carbon atom. The amino acid may be selected from the group consisting of glycine, alanine, valine, leucine, isoleucine, threonine, serine, cysteine, cystine, methionine, aspartic acid, asparagine, glutamic acid, diiodotyrosine, lysine, arginine, histidine, phenylalanine, tyrosine, tryptophan, proline, oxyproline, and combinations thereof, but not limited thereto. The term "organic acid" may be an acidic organic compound, specifically, an organic compound including a carboxyl group and a sulfone group. As an example of the organic acid, lactate, acetate, succinate, butyrate, palmitate, oxalate, tartaric acid, citrate, tartrate, propionate, hexenoic acid, capric acid, caprylic acid, valeric acid, or citric acid, but not limited thereto. In one specific embodiment, the amino acid may be L-lysine, but not limited thereto.

In the present disclosure, a microorganism before introducing a protein encoded by the foreign *ftsN* gene (for example, E. coli-derived *ftsN* gene) may be represented by "parent microorganism (or parent strain) or host cell", in order to distinguish the introduced microorganism.

In one embodiment, the host microorganism may be selected from all microorganisms having production ability of a target product. In one specific embodiment, the host microorganism may be selected from all microorganisms having production ability of L-amino acid (for example, L-lysine). In one embodiment, the host microorganism may be a microorganism naturally having L-lysine production ability, or a microorganism having L-lysine production ability by introducing mutation into a parent strain having no or significantly little L-lysine production ability.

In one embodiment, the host microorganism may be a microorganism of the genus *Corynebacterium.* The microorganism of the genus *Corynebacterium* may be *Corynebacterium glutamicum, Corynebacterium crudilactis, Corynebacterium deserti, Corynebacterium efficiens, Corynebacterium callunae, Corynebacterium stationis, Corynebacterium singulare, Corynebacterium halotolerans, Corynebacterium striatum, Corynebacterium pollutisoli, Corynebacterium imitans, Corynebacterium testudinoris* or *Corynebacterium flavescens,* but not necessarily limited thereto. Specifically, the microorganism of the genus *Corynebacterium* may be *Corynebacterium glutamicum.*

In one embodiment, the microorganism in which a protein encoded by the foreign *ftsN* gene (for example, E. coli-derived *ftsN* gene) is introduced may have increased production ability of a target product (for example, L-amino acid), compared to a homogenous non-modified microorganism, as the protein encoded by the foreign *ftsN* gene is introduced.

In one embodiment, the microorganism in which a protein encoded by the foreign *ftsN* gene (for example, E. coli-derived *ftsN* gene) is introduced may have an increased strain growth rate (viability), compared to a homogenous non-modified microorganism, as the protein encoded by the foreign *ftsN* gene is introduced.

The non-modified microorganism may refer to a microorganism which does not express the foreign cell division protein FtsN, which is a homogenous microorganism in which a protein encoded by the foreign *ftsN* gene is not introduced or a microorganism before the mutation is introduced.

The "strain growth rate" may mean the extent to which a strain survives under specific conditions, and for the purpose of the present disclosure, the strain growth rate may be interchangeably used with the terms of "rate of growth of strain", "strain viability", "cell viability", "cell growth rate", and "rate of growth of cell". In one embodiment, the strain growth rate may be determined by measuring the OD value of strain (microbial cell) cultured solution at a specific point in the culture.

Other embodiment of the present disclosure provides a method for production of a target product, comprising culturing a microorganism into which a protein encoded by the foreign *ftsN* gene is introduced in a medium.

In the method of the present disclosure, the "foreign *ftsN* gene", "protein encoded by *ftsN* gene", "microorganism", "target product" and the like are as described in the other embodiments.

In the present disclosure, the term, "culture" refers to growing the microorganism of the present disclosure (for example, microorganism of the genus *Corynebacterium)* under an artificially controlled environmental condition. The culture process of the present disclosure may be conducted according to appropriate media and culture conditions known in the art. This culture process may be easily adjusted and used by those skilled in the art depending on the selected strain. Specifically, the culture may be batch, continuous and/or fed-batch, but is not limited thereto.

In the present disclosure, the term, "medium" refers to a substance in which nutrients required for culturing the microorganism of the present disclosure (for example, microorganism of the genus *Corynebacterium)* are mixed as major components, and supplies nutrients and growth factors and the like including water essential for survival and development. Specifically, for the medium used for the culturing of the microorganism of the present disclosure and other culture condition, any one may be used without particular limitations, as long as it is a medium used for culturing a common microorganism, but the microorganism of the present disclosure may be cultured in a common medium containing suitable carbon sources, nitrogen sources, phosphorus sources, inorganic compounds, amino acids, and/or vitamins, while adjusting the temperature, pH and the like under an aerobic condition.

In the present disclosure, the carbon source may include carbohydrates such as glucose, saccharose, lactose, fructose, sucrose, maltose, etc.; sugar alcohols such as mannitol, sorbitol, etc.; organic acids such as pyruvate, lactate, citrate, etc.; amino acids such as glutamate, methionine, lysine, etc., and the like. In addition, natural organic nutrients such as starch hydrolysate, molasses, blackstrap molasses, rice bran, cassava, sugar cane residues and corn steep liquor may be used, and specifically, carbohydrates such as glucose and sterilized pre-treated molasses (i.e., molasses converted into reducing sugar) and the like may be used, and other appropriate carbon sources may be used variously without limitation. These carbon sources may be used alone or be used in combination of two or more kinds, but not limited thereto.

As the nitrogen source, inorganic nitrogen sources such as ammonia, ammonium sulfate, ammonium chloride, ammonium citrate, ammonium phosphate, ammonium carbonate, ammonium nitrate and the like; amino acids such as glutamate, methionine, glutamine and the like; and organic nitrogen sources such as amino acids such as glutamate, methionine, glutamine and the like, peptone, NZ-amine, meat extract, yeast extract, malt extract, corn steep liquor, casein hydrolysate, fish or its decomposition product, defatted soybean cake or its decomposition product and the like may be used. These nitrogen sources may be used alone or be used in combination of two or more kinds, but not limited thereto.

The phosphorus source may include monopotassium phosphate, dipotassium phosphate, or sodium-containing salts corresponding thereto and the like. As the inorganic compound, sodium chloride, calcium chloride, iron chloride, magnesium sulfate, iron sulfate, manganese sulfate, calcium carbonate and the like may be used, and other amino acids, vitamins, and/or appropriate precursors and the like may be comprised. These components or precursors may be added to a medium in a batch or continuous manner. However, it is not limited thereto.

In addition, during culture of the microorganism of the present disclosure, a compound such as ammonium hydroxide, potassium hydroxide, ammonia, phosphate, sulfate, and the like is added to a medium by an appropriate method, to adjust the pH of the medium. Furthermore, during culture, using an antifoaming agent such as fatty acid polyglycol ester, air formation may be inhibited. In addition, in order to maintain the aerobic condition of the medium, oxygen or oxygen-containing gas may be injected into the medium, or in order to maintain the anaerobic and non-aerobic condition, without injection of gas, or nitrogen, hydrogen or carbon dioxide gas may be injected, but not limited thereto.

The culture temperature in the culture of the present disclosure may be maintained at 20 to 45°C, specifically, 25 to 40°C, and the culture may be performed for about 10 to 160 hours, but not limited thereto.

The target product (for example, L-amino acid) produced by the culture of the present disclosure may be secreted into the medium or remain in cells.

The method of production of a target product (for example, L-amino acid) of the present disclosure may further comprise preparing the microorganism of the present disclosure, preparing a medium for culturing the microorganism, or a combination thereof (in any order), for example, before the culturing.

The method of production of a target product of the present disclosure may further comprise recovering the target product (for example, L-amino acid) from the medium according to the culturing (medium in which culturing is performed) or the microorganism. The recovering may be further comprised after the culturing.

The recovering may collect a targeted product (for example, L-lysine) using an appropriate method known in the art according to the culturing method of the microorganism of the present disclosure, for example, a batch, continuous or fed-batch culturing method, or the like. For example, centrifugation, filtration, treatment by a crystallized protein precipitator (salting out), extraction, ultrasonic crushing, ultrafiltration, dialysis, various kinds of chromatography such as molecular sieve chromatography (gel filtration), adsorption chromatography, ion exchange chromatography, affinity chromatography, and the like, HPLC or a combination of these methods may be used, and using an appropriate method known in the art, the targeted product (for example, L-amino acid) may be recovered from the medium or microorganism.

In addition, the method of production of a target product of the present disclosure may additionally comprise purifying. The purifying may be performed, using an appropriate method known in the art. In one embodiment, when the method of production of a target product of the present disclosure comprises both the recovering and purifying, the recovering and purifying may be continuously or non-continuously performed regardless of the order, or may be performed at the same time or by being integrated as one step, but not limited thereto.

Other embodiment of the present disclosure provides a composition for producing a target product (for example, L-amino acid) comprising the microorganism, a medium in which the microorganism is cultured, or a combination thereof.

The composition may further comprise any appropriate excipient commonly used for a composition for producing a target product, and such an excipient may be for example, a preservative, a wetting agent, a dispersant, a suspending agent, a buffer, a stabilizer or a tonicifying agent, or the like, but not limited thereto.

Other embodiment provides a use of the microorganism for using in production of a target product (for example, L-amino acid).

Other embodiment provides a use of the microorganism for using in preparation of a composition for producing a target product (for example, L-amino acid).

### [ADVANTAGEOUS EFFECTS]

The microorganism into which the activity of the cell division protein derived from E. coli of the present disclosure was confirmed to have excellent cell viability even under extreme fermentation conditions and high productivity of target products including amino acids, and thus, the microorganism can be usefully used for production of target products such as amino acids and the like.

### [MODE FOR INVENTION]

Hereinafter, the present disclosure will be described in more detail by examples. However, the following examples are merely preferable embodiments for illustrating the present disclosure, and therefore, are not intended to limit the scope of the present disclosure thereto. On the other hand, technical matters not described in the present disclosure can be sufficiently understood and easily implemented by those skilled in the technical field of the present disclosure or a similar technical field.

### Example 1. Construction of plasmid for gene introduction site deletion

In order to insert an E. coli-derived *ftsN* gene into *Corynebacterium glutamicum* chromosome, NCgl0998-1000 known as a gene encoding a transposon (Transposase) was used as an insertion site, and in order to substitute the *NCg10998-1000* gene with the E. coli *ftsN, a NCg10998-1000* deletion and target gene insertion vector was produced by the following method.

Specifically, to produce a vector, PCR was performed using the primer pair of SEQ ID NO: 1 and SEQ ID NO: 2, and SEQ ID NO: 3 and SEQ ID NO: 4, respectively, using chromosome of *Corynebacterium glutamicum* ATCC13032 as a template.

As polymerase for PCR reaction, PfuUltraTM high-fidelity DNA polymerase (Stratagene) was used, and PCR conditions were denaturation at 95°C for 30 seconds; and annealing at 55°C for 30 seconds; and polymerization at 72°C for 1 minute, and the denaturation, annealing and polymerization of the conditions were repeated 28 times. As a result, DNA fragments of 500bp and 506bp, respectively, were obtained. The obtained DNA products were purified using a PCR purification kit (QIAGEN), and the purified amplification products were treated with restriction enzyme SmaI, and then cloned using pDC24 vector (SEQ ID NO: 11) heat-treated at 65°C for 20 minutes and an infusion cloning kit (TaKaRa) according to the provided manual to construct a vector for *NCgl0998-1000* deletion and target gene insertion, pDC24_△0998-1000 vector.

The sequences of the primers used in Example 1 were shown in Table 1 below:

**[Table 1]**

| Name | Sequence (5'-> 3') | SEQ ID NO |
|---|---|---|
| Primer 1 | TCGAGCTCGGTACCCTAATAATTCTGCTTCCCGACG ATG | SEQ ID NO: 1 |
| Primer 2 | CTCTTCCGGTTCCCGGGACGGTCGATGTCGAGGCG TTACA | SEQ ID NO: 2 |
| Primer 3 | CTCGACATCGACCGTCCCGGGAACCGGAAGAGCC CGATTG | SEQ ID NO: 3 |
| Primer 4 | CTCTAGAGGATCCCCGAAAAGGAAGAAGTCTGAG AAGAAG | SEQ ID NO: 4 |

### Example 2. Production of recombinant plasmid for introduction of E. coli-derived ftsN gene

In order to introduce an *E*. coli-derived *ftsN* gene into a microorganism of the genus *Corynebacterium,* a plasmid intensifying the E. coli-derived *ftsN* gene was constructed under CJ7 promoter derived from *Corynebacterium ammoniagenes* (Korean Patent No. 10-0620092, hereinafter, Pcj7) by the following method.

Specifically, PCR was performed using a plasmid comprising the Pcj7 sequence, p117-cj7-gfp, as a template, and using the primer pair of SEQ ID NO: 5 and SEQ ID NO: 6 to obtain Pcj7 promoter fragments. In addition, PCR was performed using chromosome of E. coli as a template and using the primer pair of SEQ ID NOs: 7 and 8 to obtain *ftsN* gene DNA fragments.

As polymerase for PCR reaction, PfuUltraTM high-fidelity DNA polymerase (Stratagene) was used, and PCR conditions were denaturation at 95°C for 30 seconds; and annealing at 55°C for 30 seconds; and polymerization at 72°C for 2 minutes, and the denaturation, annealing and polymerization of the conditions were repeated 28 times. As a result, 318bp DNA fragments at the Pcj7 promoter site and 960bp DNA fragments at the *ftsN* gene site of *E. coli* were obtained, respectively.

The obtained amplification products were purified using a PCR purification kit (QUIAGEN), and used as insertion DNA fragments for vector construction. The purified amplification products were treated with restriction enzyme SmaI, and then the amplification products, the inserted DNA fragments, were cloned using pDC24_△0998-1000 vector heat-treated at 65°C for 20 minutes and an infusion cloning kit (TaKaRa) according to the provided manual to obtain a vector for introducing the *E. coli*-derived *ftsN* gene on chromosome, pDC24_△0998-1000::Pcj7_ftsN(e.co) vector.

The sequences of the primers used in Example 2 were shown in Table 2 below:

**[Table 2]**

| Name | Sequence (5'-> 3') | SEQ ID NO |
|---|---|---|
| Primer 5 | CGACATCGACCGTcccAGAAACATCCCAGCGCTACT AATAG | SEQ ID NO: 5 |
| Primer 6 | GAGTGTTTCCTTTCGTTGGGTACGT | SEQ ID NO: 6 |
| Primer 7 | CGAAAGGAAACACTCGTGGCACAACGAGATTATGT ACGCCG | SEQ ID NO: 7 |
| Primer 8 | GCTCTTCCGGTTCCCTCAACCCCCGGCGGCGAGCC GAATG | SEQ ID NO: 8 |

### Example 3. Production of E. coli-derived ftsN gene-introduced microorganism of the genus Corynebacterium and evaluation of strain growth and lysine production ability over culture time

Culture of a strain producing lysine inhibits activity of the strain due to an increase in osmotic pressure as lysine production increases, and because of this, also reduces the growth of the strain. In an environment where osmotic pressure increases, there are disadvantages that the cell viability of the strain decreases, and the membrane rigidity is weakened, so the lysine production ability of the strain becomes lower. In the present example, in order to confirm whether the viability and lysine production ability of the E. coli-derived *ftsN* gene-introduced microorganism of the genus *Corynebacterium* increase under the condition of increased osmotic pressure, the *E.* coli-derived *ftsN* gene-introduced microorganism of the genus *Corynebacterium* was produced to perform flask culture evaluation.

Specifically, the 1 kind of the vector produced in Example 2 was transformed into a lysine producing strain, *Corynebacterium glutamicum* CJ3P (US 9556463 B2) by electroporation (Van der Rest et al., Appl. Microbiol. Biotecnol. 52:541-545, 1999), and the corresponding strain was named CJ3P_*ftsN* (e.co).

In order to confirm the growth and L-lysine production ability in the medium of the produced *CJ3P_ftsN* (e.co) and the parent strain (control group), *Corynebacterium glutamicum* CJ3P, flask fermentation titer evaluation was performed by the following method.

At first, each strain was inoculated into a 250 ml corner-baffled flask containing a seed medium of 25 ml, and cultured with shaking at 200 rpm, at 37 °C for 20 hours. The seed cultured solution of 1 ml was inoculated in a 250 ml corner-baffled flask containing a production medium of 24 ml and cultured wit shaking at 200 rpm, at 37 °C for 48 hours. After completing the culture, using HPLC, the lysine concentration in the cultured solution was measured, and using this, the lysine concentration increase rate was calculated. In addition, in order to confirm the effect of introduction of the E. coli-derived *ftsN* gene on strain growth (cell viability), the *Corynebacterium glutamicum* CJ3P strain and CJ3P_*ftsN* (e.co) strain were inoculated into the production medium by the same method as the above method, and then the OD600nm value of the cultured solution diluted 20-fold was measured using a spectrophotometer every 18 hours, 36 hours and 48 hours. As a control for OD measurement, the production medium used for culture (microbial cells were not inoculated) was used. For lysine concentration measurement, high-performance liquid chromatography (HPLC, Shimadzu) was used.

### <Seed medium>

Sucrose 20 g, peptone 10 g, yeast extract 5 g, urea 1.5 g, KH₂PO4 4 g, K₂HPO₄ 8g, MgSO₄·7H₂O 0.5 g, biotin 0.1 mg, thiamine HCl 1 mg, calcium-pantothenic acid 2 mg, nicotinamide 2 mg (based on 1 liter of distilled water)

### <Production medium>

Sucrose 100 g, (NH₄)₂SO₄ 40 g, soybean protein 2.5 g, corn steep solids 5 g, urea 3 g, KH₂PO₄ 1 g, MgSO₄·7H₂O 0.5 g, biotin 100 *µ*g, thiamine hydrochloride 1000 *µ*g, calcium-pantothenic acid 2000 *µ*g, nicotinamide 3000 *µ*g, CaCO₃ 30 g (based on 1 liter of distilled water)

The experiment was repeated 3 times, and the mean value of the analysis results was shown in Table 3 below:

**[Table 3]**

| Strain | Microbial cell (OD₆₀₀ₙₘ) | | | Lysine concentration (g/L) | Lysine concentration increase rate (%) |
|---|---|---|---|---|---|
| | 18h | 36h | 48h | at 48h | |
| CJ3P | 49.1 | 71.5 | 61.5 | 7.53 | 100 |
| CJ3P_*ftsN* (e.co) | 58.5 | 81.5 | 78.1 | 8.81 | 116.9 |

As a result, as shown in Table 3, it was confirmed that the phenomenon of microbial cell reduction with increasing culture time was alleviated in the CJ3P_*ftsN* (e.co) strain into which the E. coli-derived *ftsN* gene was introduced, when the parent strain, CJ3P strain, and the *E. coli-*derived *ftsN* gene-introduced CJ3P_*ftsN* (e.co) strain were cultured, and it was confirmed that the microbial cells were also high at the 18 hours after culture.

Through the corresponding results, it could be confirmed that the introduction of the E. coli-derived *ftsN* gene could inhibit the decrease in the cell activity over culture time of the microorganism of the genus *Corynebacterium,* and the lysine production ability increased as the cell activity increased.

From the above description, those skilled in the art to which the present disclosure pertains will understand that the present disclosure can be implemented in other specific forms without changing the technical spirit or essential features thereof. In this regard, it should be understood that the examples described above are exemplary and not restrictive in all aspects. The scope of the present disclosure should be interpreted as including all changed or modified forms derived from the meaning and scope of the claims described below and equivalent concepts thereof rather than the detailed description above.

## Claims

1. A microorganism of the genus *Corynebacterium,* into which a protein encoded by an E. coli-derived *ftsN* gene is introduced.

2. The microorganism of the genus *Corynebacterium* according to claim 1, wherein the protein comprises the amino acid sequence of SEQ ID NO: 9.

3. The microorganism of the genus *Corynebacterium* according to claim 1, wherein the protein is encoded by a polynucleotide comprising the nucleic acid sequence of SEQ ID NO: 10.

4. The microorganism of the genus *Corynebacterium* according to claim 1, wherein the microorganism of the genus Corynebacterium is *Corynebacterium glutamicum.*

5. The microorganism of the genus *Corynebacterium* according to claim 1, wherein the microorganism of the genus *Corynebacterium* has increased production ability of L-lysine compared to a microorganism of the genus *Corynebacterium* into which the protein encoded by the E. coli-derived *ftsN* gene is not introduced.

6. The microorganism of the genus *Corynebacterium* according to claim 1, wherein the microorganism of the genus *Corynebacterium* has an increased strain growth rate compared to a microorganism of the genus *Corynebacterium* into which the protein encoded by the E. coli-derived *ftsN* gene is not introduced.

7. A method for producing L-lysine, comprising culturing a microorganism into which a protein encoded by an E. coli-derived *ftsN* gene is introduced in a medium.

8. The method for producing L-lysine according to claim 7, wherein the method further comprises recovering L-lysine from the cultured microorganism, medium or both of them.

9. The method for producing L-lysine according to claim 7, wherein the microorganism is a microorganism of the genus *Corynebacterium.*

10. The method for producing L-lysine according to claim 9, wherein the microorganism of the genus *Corynebacterium* is *Corynebacterium glutamicum.*
